(19) Europäisches Patentamt
European Patent Office
Office européen des brevets

(11) EP 1 720 510 B1

(12) # EUROPÄISCHE PATENTSCHRIFT

(45) Veröffentlichungstag und Bekanntmachung des Hinweises auf die Patenterteilung:
**24.07.2013 Patentblatt 2013/30**

(21) Anmeldenummer: **05701376.5**

(22) Anmeldetag: **08.02.2005**

(51) Int Cl.:
***A61K 8/18*** *(2006.01)*

(86) Internationale Anmeldenummer:
**PCT/EP2005/001244**

(87) Internationale Veröffentlichungsnummer:
**WO 2005/084631 (15.09.2005 Gazette 2005/37)**

(54) **UV-FILTER IN PUDERFORM**

UV FILTERS IN POWDER FORM

FILTRE UV SOUS FORME DE POUDRE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **03.03.2004 DE 102004010313**

(43) Veröffentlichungstag der Anmeldung:
**15.11.2006 Patentblatt 2006/46**

(73) Patentinhaber: **Merck Patent GmbH**
**64293 Darmstadt (DE)**

(72) Erfinder:
- **PFLÜCKER, Frank**
**64297 Darmstadt (DE)**
- **BECK, Jörn**
**64342 Seeheim-Jugenheim (DE)**
- **DRILLER, Hansjürgen**
**64823 Gross-Umstadt (DE)**

(56) Entgegenhaltungen:
**WO-A-03/020236**
**DE-A1- 10 163 256**
**DE-A1- 10 254 334**
**FR-A- 2 548 018**
**US-A- 5 389 361**
**US-A- 6 106 849**
**US-A1- 2002 121 227**
**US-A1- 2003 143 166**
**US-B1- 6 238 650**
**US-B1- 6 337 089**



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft UV-Filter in Puderform, erhältlich durch Sprühtrocknung einer Dispersion von verkapselten organisation UV-Filtern, wobei die Wände der Kapseln anorganisch sind, Verfahren zur Herstellung von UV-Filtern in Puderform sowie Zubereitungen enthaltend UV-Filter in Puderform.

UV-Filter spielen in einer Reihe von Anwendungen, insbesondere aber in der Kosmetik eine große Rolle. An die Hautverträglichkeit und die Anwendungseigenschaften der UV-Filter werden dabei steigende Anforderungen gestellt. Im Falle von organischen UV-Filtern soll beispielweise eine Penetration der Haut durch den UV-Filter vermieden werden, um die Belastung der Haut mit organischen Substanzen möglichst gering zu halten. Um dies zu umgehen ist z.B. in EP 1 382 328, WO 00/09652, WO 00/72806 und WO 00/71084 vorgeschlagen worden, organische UV-Filter zu verkapseln und damit zu immobilisieren. Die so verkapselten UV-Filter sind als Dispersionen zugänglich.

Dispersionen erweisen sich in einer Reihe von Anwendungen als nachteilig. Der eigentliche UV-Filter kann nicht als Reinstoff zugegeben werden, sondern immer nur in Kombination mit den Additiven, z.B. Wasser, Dispergierhilfsmitteln oder Konservierungsmitteln. Ganz besonders die Vermeidung von Konservierungsmitteln und Wasser erhöht die Quantität und Flexibilität von Formulierungen. Insbesondere in der Kosmetik erweist sich die Anwesenheit der Additive als problematisch, da kosmetische Zubereitungen vielfach ein aufeinander genau abgestimmtes Verhältnis von Feststoff zu flüssiger Phase, z.B. in Form eines Bindemittels, aufweisen.

Um beispielsweise Presspuder-Mischungen mit einem UV-Schutz zu versehen, wird ein Teil des Bindemittels üblicherweise durch flüssige UV-Filter oder durch die UV-Filterdispersion substituiert und dann dem Basisfeststoff des Puders zugesetzt. Dabei wird die Flexibilität des Binders eingeschränkt und bei Zugabe des Bindemittels zum Puder kommt es zu einer Veränderung der Mischungsverhältnisse Feststoff/Bindemittel. Dies jedoch kann zu nachteiligen Auswirkungen bezüglich der Anwendungseigenschaften führen, wie z.B. zu Farbveränderungen oder einer Verschlechterung des Hautgefühls bei topischer Applikation, was von den Anwendern unerwünscht ist.

Es bestand daher die Aufgabe, UV-Filter, insbesondere verkapselte flüssige UV-Filter in einer Form bereit zu stellen, die den direkten Einsatz der UV-Filter ermöglicht und dabei die Eigenschaften der UV-Filter, insbesondere deren Stabilität, nicht negativ beeinflusst. Letzteres gilt insbesondere für flüssige UV-Filter in verkapselter Form, deren Verkapselung dabei idealerweise erhalten bleibt, um die unerwünschte Freisetzung der in den Kapseln liegenden UV-Filter zu vermeiden. Zur Verbesserung der Anwendungseigenschaften sollen die UV-Filter in fester Form vorliegen, insbesondere ist dies für verkapselte UV-Filter erwünscht. Damit ständen für die Einarbeitung in kosmetischen Formulierungen verkapselte flüssige UV-Filter in fester Form zur Verfügung. Überraschenderweise wurde gefunden, dass diese Anforderungen durch die vorliegende Erfindung erfüllt werden.

Im Stand der Technik ist Folgendes bekannt:

US 2003/143166 A1 beschreibt unverkapselte pulverförmige UV-Filter, die durch Sprühtrocknung erhalten werden.

US 6337089 B offenbart verkapselte UV-Filter, sowie ein Verfahren zu deren Herstellung, wobei deren Kapselwände aus Organopolysiloxan bestehen.

US 6238650 B beschreibt verkapselte UV-Filter und ein Verfahren zu deren Herstellung, welche durch Gefriertrocknung erhalten werden.

Gegenstand der Erfindung sind demgemäss UV-Filter in Puderform, erhältlich durch Sprühtrocknung einer Dispersion enthaltend UV-Filter wobei die UV-Filter verkapselte organische UV-Filter sind, wobei die Wänder der Kapseln anorganisch sind. Ein weiterer Gegenstand der vorliegenden Erfindung sind Verfahren zur Herstellung der UV-Filter in Puderform, wobei Dispersionen enthaltend UV-Filter sprühgetrocknet werden. Weiterhin sind Zubereitungen enthaltend UV-Filter in Puderform ebenfalls Gegenstand der vorliegenden Erfindung.

Die erfindungsgemäßen UV-Filter in Puderform haben den Vorteil, dass sie besser handhabbar und verarbeitbar sind als Dispersionen. So lassen sich die erfindungsgemäßen Puder sowohl in wässrige als auch in nichtwässrige Phasen problemlos einarbeiten, was mit einer Dispersion nicht möglich ist. Darüber hinaus bietet sich bei den UV-Filtern in Puderform die Möglichkeit einer gezielten Modifizierung der Oberfläche, z.B. in Form einer Hydrophobierung oder Hydrophilisierung, um die anwendungstechnischen Eigenschaften nochmals zu verbessern. Da die erfindungsgemäßen Puder trocken sind, ist ein Zusatz von Konservierungsmitteln nicht nötig, was bei Dispersionen sehr wohl vonnöten ist, um eine Verkeimung der Dispersion zu verhindern. Auch die Erreichung einer Keimfreiheit durch Einstellung eines niedrigen pH-Wertes in der Dispersion, ist durchaus üblich, erweist sich aber insbesondere in der Kosmetik als problematisch, da dort der pH-Wert der Endanwendung eine besondere Rolle spielt. Dies wird bei den erfindungsgemäßen UV-Filtern vermieden, da das Puder direkt eingesetzt werden kann. Ein weiterer Vorteil der erfindungsgemäßen Puder ist die erhebliche Kompaktierung gegenüber einer Dispersion, die insbesondere die Transportkosten erheblich verringert. Durch die Entfernung des Dispersionsmittels, z.B. Wasser oder Ethanol, wird das Volumen und das Gewicht des zu

transportierenden UV-Filters erheblich verringert, was einen erheblichen positiven Einfluss auf die Transport- und Lagerkosten ausübt. Darüber hinaus kann bei Verwendung der UV-Filter in Puderform dieser Presspuder-Mischungen zugesetzt werden, wobei gleichzeitig der Binder hinsichtlich seines Hautgefühls optimiert werden kann, ohne einen zusätzlichen Anteil an flüssigen UV-Filtern berücksichtigen zu müssen.

Gegenstand der Erfindung sind UV-Filter in Puderform, erhältlich durch Sprühtrocknung einer Dispersion enthaltend UV-Filter, wobei der UV-Filter ein verkapselter organischer UV-Filter ist. Vorzugsweise handelt es sich um partikuläre UV-Filter, , wobei die Verkapselung anorganisch ist. Derartige UV-Filter sind bislang nur in Form von Dispersionen erhältlich, was den Anwendungsbereich einschränkt und die Herstellung von Zubereitungen erschwert, da das Dispersionsmittel und dessen Eigenschaften stets bei der Herstellung der Zubereitungen mit berücksichtigt werden muss. Dieser Nachteil entfällt bei den erfindungsgemäßen UV-Filtern in Puderform.

Die Größe der UV-Filter in partikulärer Form liegt im Bereich von 10 nm bis 100 μm, vorzugsweise im Bereich von 10 nm bis 30 μm ($d_{50} \leq 30$ μm, gemessen mittels Laserbeugung in Wasser, z.B. mit Malvern Particle Sizer), insbesondere im Bereich von 0.1 μm bis 20 μm.

Erfindungsgemäß werden verkapselte organische UV-Filter, eingesetzt. Im Einzelnen ergeben sich durch die Verkapselung die folgenden Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher kann es erfindungsgemäß bevorzugt sein, wenn einer oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln weisen Wände aus anorganischen Polymeren auf. Erfindungsgemäß einzusetzende Kapseln weisen Wände auf, die durch einen Sol-Gel-Prozeß, wie er in den Anmeldungen EP 1 382 328, WO 00/09652, WO 00/72806 und WO 00/71084 beschrieben ist, erhalten werden können. Bevorzugt sind hier wiederum Kapseln, deren Wände aus Kieselgel (Silica; undefiniertes Siliziumoxidhydroxid) oder Siliciumdioxid aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Für die Verkapselung geeignete UV-Filter s ind z.B. Dibenzoylmethanderivate.

Die erfindungsgemäß verwendbaren Dibenzoylmethanderivate können insbesondere unter den Dibenzoylmethanderivaten der folgenden Formel ausgewählt sein:

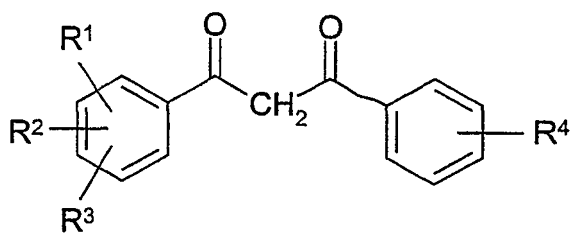

worin $R^1$, $R^2$, $R^3$ und $R^4$, die identisch oder voneinander verschieden sind, Wasserstoff, eine geradkettige oder verzweigte

$C_{1-8}$-Alkylgruppe oder eine geradkettige oder verzweigte $C_{1-8}$-Alkoxygruppe bedeuten. Gemäß der vorliegenden Erfindung können selbstverständlich ein Dibenzoylmethanderivat oder mehrere Dibenzoylmethanderivate verwendet werden. Von den Dibenzoylmethanderivaten, auf die sich die vorliegende Erfindung spezieller bezieht, können insbesondere:

- 2-Methyldibenzoylmethan,
- 4-Methyldibenzoylmethan,
- 4-Isopropyldigenzoylmethan,
- 4-tert.-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Methoxy-tert.-butyldibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan und
- 2,6-Dimethyl-4-tert.-butyl-4'-methoxydibenzoylmethan genannt werden, wobei diese Aufzählung nicht einschränkend ist.

Von den obengenannten Dibenzoylmethanderivaten wird erfindungsgemäß insbesondere das 4,4'-Methoxy-tert.-butyldibenzoylmethan und insbesondere das unter der Handelsbezeichnung Eusolex® 9020 von der Firma Merck KGaA im Handel befindliche 4,4'-Methoxy-tert.-butyldibenzoylmethan bevorzugt, wobei dieses Filter der folgenden Strukturformel entspricht:

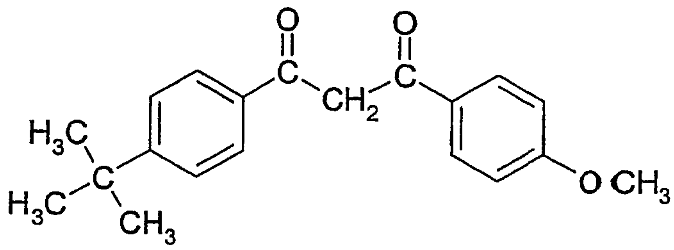

Ein weiteres erfindungsgemäß bevorzugtes Dibenzoylmethanderivat ist das 4-Isopropyldibenzoylmethan.
Die Kapseln können selbstverständlich auch einen oder mehrere Sonnenschutzfilter, die im UV-A-Bereich und/oder UV-B-Bereich und/oder IR und/oder VIS-Bereich (Absorber) wirksam sind, enthalten. Diese Filter können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben.
Prinzipiell kommen alle UV-Filter für eine Verkapselung in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B. Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL), Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000), Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS), 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25), Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure, und weitere Substanzen wie

- 2-Cyano-3,3-diphenylacrylsäure-2-ethylhexylester (z.B. Eusolex® OCR),
- 3,3'-(1,4-Phenylendimethylen)-bis-7,7-dimethyl-2-oxobicyclo-[2.2.1]hept-1-ylmethansulfonsäure sowie ihre Salze (z.B. Mexoryl® SX) und

- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B-Uvinul®UVA Plus, Fa. BASF).

Die in der Liste aufgeführten Verbindungen sind nur als Beispiele aufzufassen. Selbstverständlich können auch andere UV-Filter verwendet werden.

Weitere geeignete UV-Filter sind z.B.

- 2-(2H-Benzotriazol-2-yl)-4-methyl-6-(2-methyl-3-(1,3,3,3-tetramethyl-1-(trimethylsily(oxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] ($n \approx 60$) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1 H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® H EB),

Bevorzugte Verbindungen mit UV-filternden Eigenschaften sind 3-(4-Methylbenzyliden)-di-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-benzophenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanolaminsalze.

Bevorzugte Kapseln können auch Verbindungen der Formel I enthalten,

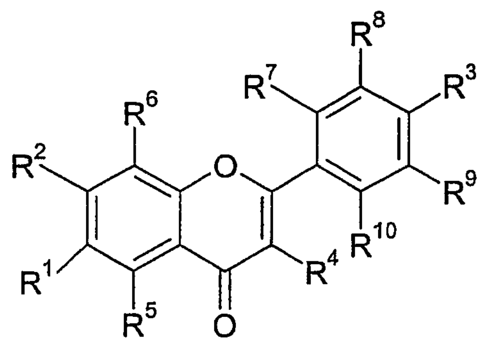

I

wobei $R^1$ und $R^2$ ausgewählt sind aus

- H
- und $OR^{11}$, wobei $OR^{11}$ unabhängig voneinander steht für
    - OH
    - geradkettige oder verzweigte $C_1$- bis $C_{20}$-Alkyloxygruppen,
    - geradkettigen oder verzweigten $C_3$- bis $C_{20}$-Alkenyloxygruppen,
    - geradkettigen oder verzweigten $C_1$- bis $C_{20}$-Hydroxyalkoxy-gruppen, wobei die Hydroxygruppe(n) an primäre oder sekundäre Kohlenstoffatome der Kette gebunden sein können und weiter die Alkylkette auch durch Sauerstoff unterbrochen sein kann, und/oder
    - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
    - Mono- und/oder Oligoglycosylreste,

mit der Maßgabe, dass mindestens ein Rest aus $R^1$ und $R^2$ steht für $OR^{11}$,
und $R^3$ steht für einen Rest $OR^{11}$ und
$R^4$ bis $R^7$ und $R^{10}$ gleich oder verschieden sein können, und unabhängig voneinander stehen für

- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenyl-gruppen, wobei die Ringe jeweils auch durch $-(CH_2)_n$-Gruppen mit n = 1 bis 3 überbrückt sein können und

$R^8$ und $R^9$ gleich oder verschieden sein können, und unabhängig voneinander stehen für

- H
- $OR^{11}$
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenyl-gruppen, wobei die Ringe jeweils auch durch $-(CH_2)_n$-Gruppen mit n = 1 bis 3 überbrückt sein können.

Unter den erfindungsgemäß einzusetzenden Flavonoiden der Formel I finden sich dabei Breitband-UV-Filter, andere ebenfalls bevorzugte Verbindungen der Formel I zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern. Bevorzugte erfindungsgemäße Kapseln enthalten zumindest eine Verbindung der Formel I, wobei $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste und

$R^1$ und/oder $R^2$ vorzugsweise stehen für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste.

Diese bevorzugten Verbindungen zeichnen sich durch eine besonders intensive UV-Absorption aus. Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy.

Selbstverständlich können in den Kapseln die oben genannten UV-Filter allein vorliegen, es können aber auch Mischungen mehrerer der genannten UV-Filter in den Kapseln sein. Darüber hinaus können die UV-Filter in den Kapseln auch kombiniert mit weiteren Substanzen, wie z.B. Photostabilisatoren, kosmetischen Ölen und/oder Antioxidantien, vorliegen, um eine Erhöhung der Stabilität der genannten UV-Filter zu erzielen. Beispiele und bevorzugte Verbindungen für die oben genannten weiteren Substanzen, insbesondere für die Photostabilisatoren finden sich im weiteren Verlauf dieser Anmeldung bei der allgemeinen Beschreibung dieser Substanzen. Als kosmetische Öle eignen sich beispielsweise Mineralöle, Mineralwachse, Öle, wie Triglyceride der Caprin- oder der Caprylsäure, ferner natürliche Öle wie z. B. Rizinusöl; Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, Silikonöle, wie z.B. Dimethylpolysiloxane, Diethylpolysiloxane, Diphenylpolysiloxane sowie Mischformen daraus.

Für die oben genannten verkapselten UV-Filter ist die Puderform von besonderem Interesse, da UV-Filter in Kapselform zumeist nur als Dispersion angeboten werden können, die die oben genannten Nachteile in den Applikationen aufweisen. Weiterhin liegen die verkapselten UV-Filter vielfach als Ethanol-haltige Dispersionen vor, was das Anwendungsgebiet zusätzlich einschränkt, da Ethanol in vielen Anwendungen, insbesondere in der Kosmetik, als Bestandteil unerwünscht ist. Die erfindungsgemäßen UV-Filter in Puderform sind vorzugsweise lösemittelfrei bzw. weisen einen in

den jeweiligen Anwendungen nicht störenden Anteil an Lösemittel auf und sind damit universell einsetzbar. Aus diesem Grund erweisen sich die erfindungsgemäßen UV-Filter in Puderform als besonders vorteilhaft.

Die erfindungsgemäßen Puder können zusätzlich in einer weiteren Ausführungsform der vorliegenden Erfindung nachbehandelt werden, um die Oberfläche der einzelnen Partikel des Puders zu modifizieren. So kann durch die Aufbringung entsprechender Verbindungen eine Hydrophobisierung oder Hydrophilisierung der Partikeloberflächen erzielt werden. Zur hydrophoben Modifikation eignet sich beispielsweise eine Beschichtung mit organischen Säuren, wie z.B. Stearinsäure oder Laurinsäure, mit LCST-Polymeren, organischen Fluoralkoholphosphaten oder eine Silicon- oder Silan-Beschichtung.

Die Silicone sind bekanntlich silizium-organische Polymere oder Oligomere mit geradkettiger oder cyclischer, verzweigter oder vernetzter Struktur mit unterschiedlichen Molekülgewichten, die durch Polymerisation und/oder Polykondensation mit geeignet funktionalisierten Silanen erhalten werden und im wesentlichen aus wiederkehrenden Haupteinheiten gebildet werden, in denen die Siliziumatome über Sauerstoffatome miteinander verknüpft sind (Siloxanbindung), wobei gegebenenfalls substituierte Kohlenwasserstoffgruppen über ein Kohlenstoffatom direkt an die Siliziumatome gebunden sind. Die gebräuchlichsten Kohlenwasserstoffgruppen sind Alkylgruppen und insbesondere Methylgruppen, Fluoralkylgruppen, Arylgruppen und insbesondere Phenylgruppen sowie Alkenylgruppen und insbesondere Vinylgruppen. Weitere Typen von Gruppen, die entweder direkt oder über eine Kohlenwasserstoffgruppe an die Siloxankette gebunden werden können, sind insbesondere Wasserstoff, die Halogene und insbesondere Chlor, Brom oder Fluor, die Thiole, Alkoxygruppen, Polyoxyalkylengruppen (oder Polyether) und insbesondere Polyoxyethylen und/oder Polyoxypropylen, Hydroxygruppen oder Hydroxyalkylgruppen, gegebenenfalls substituierte Aminogruppen, Amidgruppen, Acyloxygruppen oder Acyloxyalkylgruppen, Hydroxyalkylaminogruppen oder Aminoalkylgruppen, quaternäre Ammoniumgruppen, amphotere Gruppen oder Betaingruppen, anionische Gruppen, wie Carboxylate, Thioglykolate, Sulfosuccinate, Thiosulfate, Phosphate und Sulfate, wobei diese Aufzählung selbstverständlich in keiner Weise einschränkend ist (sogenannte 'organomodifizierte' Silicone).

Im Sinne der vorliegenden Erfindung sollen mit dem Ausdruck 'Silicone' auch die zu ihrer Herstellung benötigten Silane und insbesondere die Alkylsilane eingeschlossen und abgedeckt sein.

Die für die vorliegende Erfindung geeigneten Silicone, die zum Umhüllen der UV-Schutzmittel verwendet werden können, sind vorzugsweise unter den Alkylsilanen, den Polydialkylsiloxanen und den Polyalkylhydrogensiloxanen ausgewählt. Noch bevorzugter sind die Silicone unter Octyltrimethylsilan, den Polydimethylsiloxanen und den Polymethylhydrogenosiloxanen ausgewählt.

Weiterhin sind Gegenstand der vorliegenden Erfindung Verfahren zur Herstellung von UV-Filtern in Puderform, wie zuvorbeschrieben wobei Dispersionen von verkapselten organischen UV-Filtern sprühgetrocknet werden. Neben den beschriebenen Sprühtrocknungsvarianten, kann auch die Wirbelschichttrocknung (Wirbelschichtgranulation) zum Einsatz kommen. Des weiteren können alle nach dem Stand der Technik beschriebenen Verfahren zur schonenden Trocknung von Suspensionen verwendet werden. Erfindungsgemäß wird die Sprühtrocknung eingesetzt. Die geeigneten Arten von UV-Filter sind bereits bei der Beschreibung der erfindungsgemäßen Puder genannt.

Die erfindungsgemäßen Verfahren haben den Vorteil, dass auf schonende Weise UV-Filter in Puderform aus entsprechenden Dispersionen hergestellt werden können. Insbesondere bei den verkapselten organischen UV-Filtern erweist sich die Sprühtrocknung als besonders geeignetes Verfahren zur Herstellung entsprechender Puder. Die Kapseln sind empfindlich gegenüber mechanischer und starker thermischer Belastung, die zu einem Aufbrechen der Verkapselung und zu einer Freisetzung der darin eingeschlossenen Substanzen führt. Dies ist unerwünscht, da gerade bei topischen Anwendungen von UV-Filtern ein direkter Kontakt der UV-Filter mit der Haut vermieden werden soll. Der strukturelle Erhalt der Verkapselung ist damit von grundlegender Bedeutung und stellt eine wesentliche Herausforderung für diese Systeme dar. Durch die vorzugsweise in der vorliegenden Erfindung angewandte Technik der Sprühtrocknung konnte überraschenderweise diese Anforderung auch für diese Systeme erfüllt werden, so dass es somit gelungen ist, UV-Filter jeglicher Art als Puder bereitzustellen, was insbesondere bei verkapselten Systemen einen grundlegenden Vorteil darstellt.

Die jeweiligen UV-Filter werden in Form von Dispersionen in die Sprühtrocknung eingebracht. Dabei eignen sich grundsätzlich alle Lösemittel als Dispersionsmittel, wie z.B. Wasser oder organische Lösemittel. Vorzugsweise handelt es sich um wässrige Dispersionen, da hier keine unerwünschten Lösemittelrückstände in den Pudern zurückbleiben können, die den Einsatz der Puder in der Kosmetik möglicherweise erschweren oder ganz verhindern. Darüber hinaus sind bei wässrigen Dispersionen keine sicherheitsrelevanten Beschränkungen wie z.B. bezüglich der Brennbarkeit oder der Explosionsgefahr gegeben.

Für die Sprühtrocknung oder Gefriertrocknung eignen sich alle dem Fachmann bekannten Verfahrensvarianten und alle dazu geeigneten Apparaturen, vorzugsweise wird die Sprühtrocknung eingesetzt. Grundsätzlich beinhaltet der Sprühtrocknungsprozeß immer vier Grundschritte, nämlich die Tropfenerzeugung, die Tropfen/Gas-Mischung, die Abtrennung der Agglomerate und die Abscheidung des Feingutes. Hieraus ergeben sich Verfahrensvarianten bei den einzelnen Teilschritten. Die Tropfenerzeugung kann beispielsweise durch Zentrifugalzerstäubung, Einstoffdüsenzerstäubung oder Zweistoffdüsenzerstäubung erfolgen, bei der Produkt/Gas-Führung kann es sich um eine Gleichstromführung oder eine Gegenstromführung handeln. In der vorliegenden Erfindung werden bevorzugt die Zentrifugalzerstäubung und die Zweistoffdüsenzerstäubung eingesetzt, wobei es sich um eine Gleich- oder Gegenstromführung handeln kann. Insbesondere bevorzugt ist die Verwendung der Zweistoffdüsenzerstäubung im Gegenstrombetrieb oder die Zentrifugalzerstäubung in Gleichstromführung. Der Produktaustrag kann ebenfalls nach allen dem Fachmann bekannten Verfahrensvarianten erfolgen, vorzugsweise handelt es sich um eine Zweipunktabscheidung, das heißt, es wird z.B. ein Trocknerkonus und ein Zyklon verwendet.

Die Eintrittstemperatur für wässrige Dispersionen kann im Bereich von 110°C bis 200°C, vorzugsweise im Bereich von 130°C bis 160°C, und insbesondere im Bereich von 140°C bis 150°C liegen. Die Austrittstemperatur für das Puder kann im Bereich von 40 bis 90°C, vorzugsweise im Bereich von 50 bis 80°C liegen. Werden Lösemittelhaltige Dispersionen eingesetzt, so können die Eintritts- und Austrittstemperaturen entsprechend niedriger gewählt werden und damit individuell an das Lösemittel angepasst werden. Das Verhältnis von Sprühgutdurchsatz zu Trocknungsluftmenge kann im Bereich von 0.5kg/h zu 200 $m^3$/h bis 5kg/h zu 50$m^3$/h liegen, vorzugsweise liegt das Verhältnis zwischen 0.8kg/h zu 70$m^3$/h bis 1.5kg/h zu 75$m^3$/h.

In den erfindungsgemäßen Verfahren können zur Veränderung der Produkteigenschaften, sowie zur Veränderung der Sprüheigenschaften vor oder während der Sprühtrocknung oder Gefriertrocknung entsprechende Additive eingebracht werden, z.B. als Zusatz in den eingesetzten Dispersionen oder direkt als Additiv während des Verfahrens. Hierbei können verschiedenste, dem Fachmann bekannte, Hilfsstoffe wie. Zuckeralkohole, Polyole, Maisstärke, Cycylodextrine, Emulgatoren, Tenside, Cellulose-Derivate, Xantan Gum, PVP zum Einsatz kommen. Die Menge der zugesetzten Additive kann 0.01-20 Gew.-%, vorzugsweise 0.1-10 Gew.-% betragen.

In einer weiteren Ausführungsform werden die UV-Filter in Pulverform nachbehandelt. Die Nachbehandlung kann nach allen dem Fachmann bekannten Arten erfolgen, wie z.B. durch Polymerpräzipitationsverfahren in wässrigen oder organischen Medien, durch Solvolyseverfahren, durch Dispergierverfahren oder durch einfaches Mischen.

Die erfindungsgemäßen UV-Filter in Puderform eignen sich grundsätzlich zum Einsatz in jeglicher Form von Zubereitungen, wie z.B. kosmetischen Zubereitungen aber auch in Zubereitungen, die im technischen Sektor eingesetzt werden können, wie z.B. Farben oder Lacken. Bevorzugt werden die erfindungsgemäßen UV-Filter in Puderform in Zubereitungen in der Kosmetik eingesetzt, insbesondere in der dekorativen Kosmetik. Die erfindungsgemäßen UV-Filter in Puderform erlauben die Herstellung von Zubereitungen mit Lichtschutzeigenschaften für eine Vielzahl von Applikationsvarianten und -medien, die mit den aus dem Stand der Technik bekannten Dispersionen nur schwer herstellbar sind, da das Dispergiermittel die Zusammensetzung der Zubereitungen erheblich verändert. So lassen sich nach den erfindungsgemäßen Verfahren UV-Filter bereitstellen, die für den Einsatz in Presspudern geeignet sind, ohne das Hautgefühl oder die Zubereitungsmöglichkeiten zu verändern oder zu begrenzen. Der Anteil der UV-Filter in Puderform in diesen Zubereitungen kann 0.1 bis 30 Gew.-% und vorzugsweise 0.5 bis 15 Gew.-%, bezogen auf die Zubereitung, betragen. Die erfindungsgemäßen UV-Filter in Puderform können sowohl in die wässrige als auch in die ölige Phase einer Zubereitung eingearbeitet werden.

Bevorzugte Zubereitungen mit Lichtschutzeigenschaften enthalten dabei zusätzlich mindestens einen weiteren organischen UV-Filter, vorzugsweise ein Dibenzoylmethanderivat. Die im Rahmen der vorliegenden Erfindung verwendeten Dibenzoylmethanderivate sind an sich bereits wohlbekannte Produkte, die insbesondere in den Druckschriften FR-A-2 326 405, FR-A-2 440 933 und EP-A-0 114 607 beschrieben sind.

Die erfindungsgemäß verwendbaren Dibenzoylmethanderivate können insbesondere unter den Dibenzoylmethanderivaten der folgenden Formel ausgewählt sein:

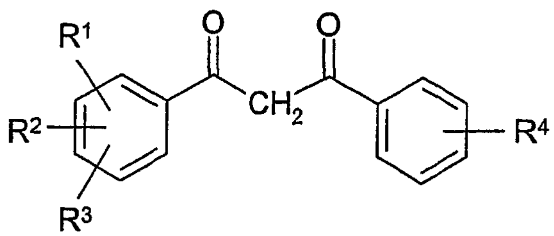

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Gemäß der vorliegenden Erfindung können selbstverständlich ein Dibenzoylmethanderivat oder mehrere Dibenzoylmethanderivate verwendet werden. Von den Dibenzoylmethanderivaten, auf die sich die vorliegende Erfindung spezieller bezieht, können insbesondere:

- 2-Methyldibenzoylmethan,
- 4-Methyldibenzoylmethan,
- 4-Isopropyldigenzoylmethan,

- 4-tert.-Butyldibenzoylmethan,
- 2,4-Dimethyldibenzoylmethan,
- 2,5-Dimethyldibenzoylmethan,
- 4,4'-Diisopropyldibenzoylmethan,
- 4,4'-Methoxy-tert.-butyldibenzoylmethan,
- 2-Methyl-5-isopropyl-4'-methoxydibenzoylmethan,
- 2-Methyl-5-tert.-butyl-4'-methoxydibenzoylmethan,
- 2,4-Dimethyl-4'-methoxydibenzoylmethan
  und
- 2,6-Dimethyl-4-tert.-butyl-4'-methoxydibenzoylmethan

genannt werden, wobei diese Aufzählung nicht einschränkend ist.

Von den obengenannten Dibenzoylmethanderivaten wird erfindungsgemäß insbesondere das 4,4'-Methoxy-tert.-butyldibenzoylmethan und insbesondere das unter der Handelsbezeichnung Eusolex® 9020 von der Firma Merck KGaA im Handel befindliche 4,4'-Methoxy-tert.-butyldibenzoyl-methan bevorzugt, wobei dieses Filter der folgenden Strukturformel entspricht:

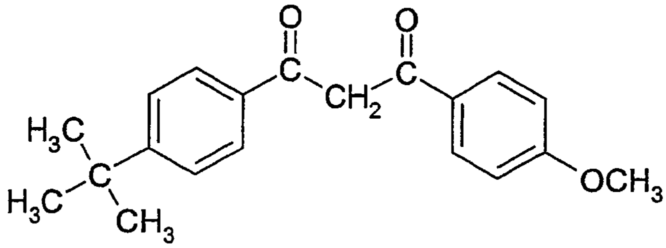

Ein weiteres erfindungsgemäß bevorzugtes Dibenzoylmethanderivat ist das 4-Isopropyldibenzoylmethan.

Weitere bevorzugte Zubereitungen mit Lichtschutzeigenschaften enthalten dabei mindestens ein Benzophenon oder Derivat des Benzophenon, wie insbesondere bevorzugt 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40).

Das oder die Dibenzoylmethanderivat(e) oder das oder die Benzophenonderivat(e) können in den erfindungsgemäßen Zusammensetzungen in Mengenanteilen vorliegen, die im Allgemeinen im Bereich von 0.1 bis 10 Gew.-% liegen und vorzugsweise in Mengenanteilen, die im Bereich von 0.3 bis 5 Gew.-% liegen, wobei diese Mengenanteile auf das Gesamtgewicht der Zusammensetzung bezogen sind.

Aufgrund der oben genannten Vorteile ist ein weiterer Gegenstand der vorliegenden Erfindung auch die Verwendung eines UV-Filters in Puderform zur Vermeidung der Destabilisierung von anderen UV-Filtern, insbesondere Dibenzoylmethan und Derivaten des Dibenzoylmethan bzw. Benzophenon und Derivaten des Benzophenon.

Weiter kann es erfindungsgemäß bevorzugt sein, wenn die Zubereitungen weitere anorganische UV-Filter enthalten. Hierbei sind sowohl solche aus der Gruppe der Titandioxide. wie z.B. gecoatetes Titandioxid (z.B. Eusolex® T-2000, Eusolex®T-AQUA), Zinkoxide (z.B. Sachtotec®), Eisenoxide oder auch Ceroxide bevorzugt. Diese anorganischen UV-Filter werden in der Regel in einer Menge von 0.5 bis 20 Gewichtsprozent, vorzugsweise 2-10 %, in kosmetische Zubereitungen eingearbeitet. Insbesondere kann es dabei bevorzugt sein, wenn in Emulsionen in eine Phase ein erfindungsgemäßer UV-Filter in Puderform und in der anderen Phase ein weiterer anorganischer UV-Filter eingearbeitet wird.

Erfindungsgemäß können die oben genannten UV-Filter auch mit einer die hydrophilen oder die hydrophoben Eigenschaften verstärkenden Oberflächenbehandlung versehen sein. Beispiele für derartige Oberflächenbehandlungen sind bereits genannt.

Die UV-Schutzmittel können in den erfindungsgemäßen Zusammensetzungen in Mengenanteilen vorliegen, die im allgemeinen im Bereich von 0.1 bis 50 Gew.-% liegen und vorzugsweise in Mengenanteilen, die im Bereich von 0.5 bis 20 Gew.-% liegen, wobei diese Mengenanteile auf das Gesamtgewicht der Zusammensetzung bezogen sind.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

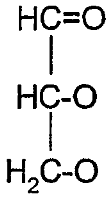 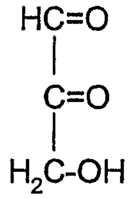 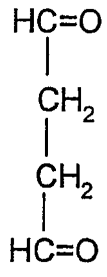 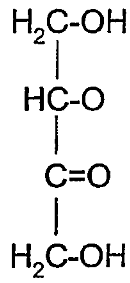

Glycerolaldehyd Hydroxymethylglyoxal γ-Dialdehyd Erythrulose

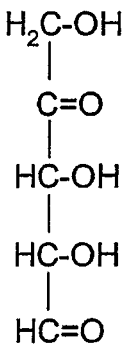 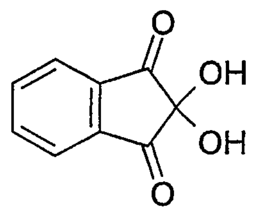

6-Aldo-D-Fructose Ninhydrin

Ferner ist das 5-Hydroxy-1,4-naphtochinon (Juglon) zu nennen, das aus den Schalen frischer Walnüsse extrahiert wird

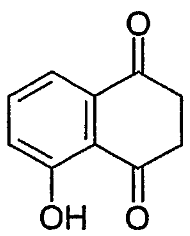

5-Hydroxy-1,4-naphtochinon (Juglon)

sowie das in den Henna-Blättern vorkommende 2-Hydroxy-1,4-naphtochinon (Lawson).

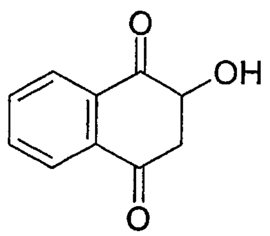

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

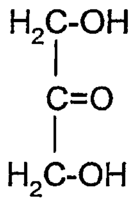

1,3-Dihydroxyaceton (DHA)

Die Verwendung eines erfindungsgemäßen UV-Filters in Puderform bei der Stabilisierung von Selbstbräunern, insbesondere Dihydroxyaceton oder Dihydroxyacetonderivaten ist ein weiterer Gegenstand der vorliegenden Erfindung.

Ferner können die erfindungsgemäßen Zubereitungen mit Lichtschutzeigenschaften auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxynaphthalin-3-carbonsäure | 15865 | Rot |
| 1-(2-Sulfo-1-naphthylazo)-2-hydroxynaphthalin-3-carbonsäure | 15880 | Rot |

(fortgesetzt)

| Chemische oder sonstige Bezeichnung | CIN | Farbe |
|---|---|---|
| 1-(3-Sulfo-1-phenylazo)-2-naphthol-6-sulfosäure | 15980 | Orange |
| 1-(4-Sulfo-1-phenylazo)-2-naphthol-6-sulfosäu re | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-β-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violet |
| Basic Violet 2 | 42520 | Violet |
| 2'-Methyl-4'-(N-ethyl-N-m-sulfobenzyl)-amino-4"-(N-diethyl)-amino-2-methyl-N-ethylIN-m-sulfobenzyl-fuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |

(fortgesetzt)

| Chemische oder sonstige Bezeichnung | CIN | Farbe |
|---|---|---|
| 3-(2'-Methylphenylamino)-6-(2'-methyl-4'-sulfophenylamino)-9-(2"-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |

(fortgesetzt)

| Chemische oder sonstige Bezeichnung | CIN | Farbe |
|---|---|---|
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen:

2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfonsäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, β-Carotin oder Cochenille.

Vorteilhaft im Sinne der vorliegenden Erfindung sind ferner Gelcrèmes mit einem Gehalt an Effektpigmenten. Bevorzugt sind insbesondere die im folgenden aufgelisteten Arten von Effektpigmenten:

1. Natürliche Effektpigmente, wie z. B.

   a) "Fischsilber" (Guanin/Hypoxanthin-Mischkristalle aus Fischschuppen) und
   b) "Perlmutt" (vermahlene Muschelschalen)

2. Monokristalline Effektpigmente, wie z.B. Bismutoxychlorid (BiOCl)
3. Schicht-Substrat Pigmente: z. B. Glimmer/Metalloxid

Basis für Effektpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Effektpigmentarten auf Basis von Glimmer/Metalloxid:

| Gruppe | Belegung/Schichtdicke | Farbe |
|---|---|---|
| **Silberweiße Effektpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck KGaA unter den Handelsnamen Timiron®, Colorona® oder Dichrona® erhältlichen Perlglanzpigmente.

Die Liste der genannten Effektpigmente soll selbstverständlich nicht limitierend sein. Im Sinne der vorliegenden Erfindung vorteilhafte Effektpigmente sind auf zahlreichen, an sich bekannten Wegen erhältlich. Darüber hinaus lassen sich beispielsweise auch andere Substrate außer Glimmer mit weiteren Metalloxiden beschichten, wie z. B. Silica und dergleichen mehr. Vorteilhaft sind z.B. mit $TiO_2$ und $Fe_2O_3$ beschichtete $SiO_2$-Partikel ("Ronaspheren"), die von der Firma Merck KGaA vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Effektpigmente, welche unter der Verwendung von $SiO_2$ hergestellt werden. Solche Pigmente, die auch zusätzlich goniochromatische Effekte haben können, sind z.B. unter dem Handelsnamen Colorstream® bei der Firma Merck KGaA erhältlich.

Weiterhin vorteilhaft können Pigmente der Firma Engelhard auf Basis von Calcium Natrium Borosilikat, die mit Titandioxid beschichtet sind, eingesetzt werden. Diese sind unter dem Namen Reflecks® erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes® Standard/Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0.1 Gew.-% bis 30 Gew.-%, vorzugsweise von 0.5 bis 15 Gew.-%, insbesondere von 1.0 bis 10 Gew.-% gewählt, jeweils bezogen auf das Gesamtgewicht der Zubereitungen.

Die erfindungsgemäßen Zubereitungen mit Lichtschutzeigenschaften können selbstverständlich einen oder mehrere zusätzliche(n) hydrophile(n) oder lipophile(n) Sonnenschutzfilter, die im UV-A-Bereich und/oder UV-B-Bereich und/oder IR und/oder VIS-Bereich (Absorber) wirksam sind, enthalten. Diese zusätzlichen Filter können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben.

Prinzipiell kommen alle UV-Filter für eine Kombination mit den in den erfindungsgemäßen Pudern enthaltenden UV-Schutzmitteln in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B. Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL), Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000), Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS), 4-Aminobenzoesäure und Derivate wie 4-Aminobenzoesäure, 4-(Dimethylamino)benzoesäu re-2-ethylhexylester (z.B. Eusolex® 6007), ethoxylierter 4-Aminobenzoesäureethylester (z.B. Uvinul® P25), Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure, und weitere Substanzen wie

- 2-Cyano-3,3-diphenylacrylsäure-2-ethylhexylester (z.B. Eusolex® OCR),
- 3,3'-(1,4-Phenylendimethylen)-bis-7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden. Insbesondere können vorteilhaft auch organische partikuläre UV-Filter, wie Sie beispielsweise in der Patentanmeldung WO 99/66896 beschrieben sind, mit den erfindungsgemäßen Pudern kombiniert werden.

Diese organischen UV-Filter werden in der Regel in einer Menge von 0.5 bis 20 Gewichtsprozent, vorzugsweise 1-10 Gew.-%, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)ph enol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5% methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Bevorzugte Verbindungen mit UV-filternden Eigenschaften sind 3-(4'-Methylbenzyliden)-di-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-benzophenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanolaminsalze.

Bevorzugte Zubereitungen können auch Verbindungen der Formel I enthalten,

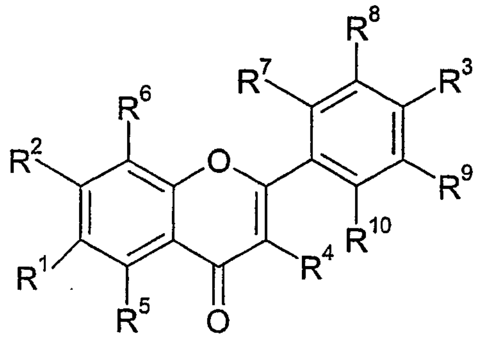

I

wobei $R^1$ und $R^2$ ausgewählt sind aus

- H
- und $OR^{11}$, wobei $OR^{11}$ unabhängig voneinander steht für
    - OH
    - geradkettige oder verzweigte $C_1$- bis $C_{20}$-Alkyloxygruppen,
    - geradkettigen oder verzweigten $C_3$- bis $C_{20}$-Alkenyloxygruppen,
    - geradkettigen oder verzweigten $C_1$- bis $C_{20}$-Hydroxyalkoxy-gruppen, wobei die Hydroxygruppe(n) an primäre oder sekundäre Kohlenstoffatome der Kette gebunden sein können und weiter die Alkylkette auch durch Sauerstoff unterbrochen sein kann, und/oder
    - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
    - Mono- und/oder Oligoglycosylreste,

mit der Maßgabe, dass mindestens ein Rest aus $R^1$ und $R^2$ steht für $OR^{11}$,
und $R^3$ steht für einen Rest $OR^{11}$ und
$R^4$ bis $R^7$ und $R^{10}$ gleich oder verschieden sein können, und unabhängig voneinander stehen für

- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenyl-gruppen, wobei die Ringe jeweils auch durch $-(CH_2)_n$-Gruppen mit n = 1 bis 3 überbrückt sein können und

$R^8$ und $R^9$ gleich oder verschieden sein können, und unabhängig voneinander stehen für

- H
- $OR^{11}$
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenyl-gruppen, wobei die Ringe jeweils auch durch $-(CH_2)_n$-Gruppen mit n = 1 bis 3 überbrückt sein können.

Vorteile der erfindungsgemäßen Zusammensetzungen sind dabei insbesondere die UV-Licht filternde Wirkung und die gute Hautverträglichkeit. Zusätzlich sind die hier beschriebenen Verbindungen der Formel I farblos oder nur schwach gefärbt und führen so, im Gegensatz zu vielen bekannten natürlich vorkommenden Flavonoiden, nicht zu Verfärbungen der Zubereitungen.

Unter den erfindungsgemäß einzusetzenden Flavonoiden der Formel I finden sich dabei Breitband-UV-Filter, andere ebenfalls bevorzugte Verbindungen der Formel I zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern. Bevorzugte erfindungsgemäße Zubereitungen mit Lichtschutzeigenschaften enthalten zumindest eine Verbindung der Formel I, wobei $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste und

$R^1$ und/oder $R^2$ vorzugsweise stehen für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste.

Diese bevorzugten Verbindungen zeichnen sich durch eine besonders intensive UV-Absorption aus.

Zusätzlich haben solche bevorzugten Verbindungen Vorteile bei der Einarbeitung in die Zubereitungen:

- Mono- und/oder Oligoglycosylreste verbessern die Wasserlöslichkeit der erfindungsgemäß einzusetzenden Verbindungen;
- geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen;

d.h. über die geeignete Auswahl der Substituenten kann die Hydrophilie bzw. Lipophilie der Verbindungen nach Formel I gesteuert werden. Als Mono- oder Oligosaccharidreste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können $\alpha$- oder $\beta$-glycosidisch mit dem Grundkörper verbunden sein. Ein bevorzugtes Disaccharid ist beispielsweise das 6-O-(6-deoxy-$\alpha$-L-mannopyranosyl)-$\beta$-D-glucopyranosid.

Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy. Daher sind Zubereitungen mit Lichtschutzeigenschaften enthaltend zumindest eine Verbindung der Formel I, die dadurch gekennzeichnet ist, dass $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy, erfindungsgemäß besonders bevorzugt. Insbesondere ist es dabei bevorzugt, wenn und $R^8$ und $R^9$ für H stehen.

Die Verbindungen der Formel I werden erfindungsgemäß typisch in Mengen von 0.01 bis 20 Gew.-%, vorzugsweise in Mengen von 0.5 Gew.-% bis 10 Gew.-% und insbesondere bevorzugt in Mengen von 1 bis 8 Gew.-% eingesetzt. Dabei bereitet es dem Fachmann keinerlei Schwierigkeiten, die Mengen abhängig von dem beabsichtigten Lichtschutzfaktor der Zubereitung entsprechend auszuwählen.

Durch Kombination von einem oder mehreren nanopartikulären UV-Schutzmitteln in den erfindungsgemäßen Pudern mit weiteren UV-Filtern kann die Schutzwirkung gegen schädliche Einwirkungen der UV-Strahlung optimiert werden. Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten.

Alle genannten UV-Filter einschließlich der Verbindungen der Formel I können ebenfalls auch in verkapselter Form eingesetzt werden. Insbesondere ist es von Vorteil organische UV-Filter in verkapselter Form einzusetzen. Beispiele für die Verkapselung sind vorab bei der Beschreibung der erfindungsgemäßen UV-Filter in Puderform bereits genannt. Darüber hinaus können auch diese Kapseln nachbehandelt se in, das heißt die Oberfläche der Partikel ist hydrophobisiert oder hydrophilisiert. Beispiele für derartige Nachbehandlungen sind ebenfalls bereits genannt.

Weisen die erfindungsgemäßen Zubereitungen Verbindungen entsprechend Formel I mit freien Hydroxy-Gruppen auf, so zeigen sie neben den beschriebenen Eigenschaften zusätzlich eine Wirkung als Antioxidans und/oder Radikalfänger. Bevorzugt sind daher auch Zubereitungen mit Lichtschutzeigenschaften enthaltend zumindest eine Verbindung der Formel I, die dadurch gekennzeichnet ist, dass mindestens einer der Reste $R^1$ bis $R^3$ steht für OH, wobei vorzugsweise mindestens einer der Reste $R^1$ oder $R^2$ für OH steht.

Damit die Verbindungen der Formel I ihre positive Wirkung als Radikalfänger auf die Haut besonders gut

entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert. Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen. Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel I zur Herstellung entsprechend einsetzbarer Zubereitungen sind ausdrücklich auch Gegenstand der vorliegenden Erfindung.

Insbesondere eignen sich bevorzugte erfindungsgemäße Zusammensetzungen auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicä, der polymorphen Akne, der Akne rosaceae, der nodulären Akne, der Akne conglobata, der alters-bedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der licht-bedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz-/Kreislauf-Erkran-kungen, wie Arteriosklerose oder Bluthochdruck, sowie des Insulinunabhängigen Diabetes, zur Behandlung von Hautproblemen, die durch UV-Strahlung hervorgerufen werden.

Die schützende Wirkung erfindungsgemäßer Zubereitungen gegen oxidativen Stress bzw. gegen die Einwirkung von Radikalen kann also weiter verbessert werden, wenn die Zubereitungen ein oder mehrere Antioxidantien enthalten, wobei es dem Fachmann keinerlei Schwierigkeiten bereitet, geeignete schnell oder zeitverzögert wirkende Antioxidantien auszuwählen.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, dadurch gekennzeichnet, dass sie neben den einer oder mehreren Verbindungen nach Formel I ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formel I in solchen Zusammensetzungen üblicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden dabei mit Verbindungen der Formel I üblicherweise in Verhältnissen im Bereich von 1000:1 bis 1:1000, bevorzugt in Mengen von 100: 1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31 (7), 869-881 untersuchen die pH-Abhängigkeit der antioxidanten Wirkung von Hydoxyflavonen. Über den gesamten pH-Bereich zeigt Quercetin die höchste Aktivität der untersuchten

Strukturen.

Geeignete Antioxidantien sind weiter Verbindungen der Formel II

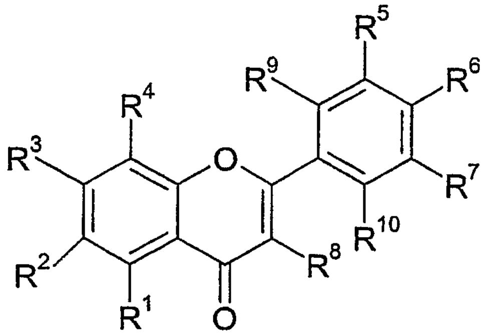

II

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für
- OH
- geradkettige oder verzweigte $C_1$- bis $C_{20}$-Alkyloxygruppen,
- geradkettigen oder verzweigten $C_3$- bis $C_{20}$-Alkenyloxygruppen,
- geradkettigen oder verzweigten $C_1$- bis $C_{20}$-Hydroxyalkoxygruppen, wobei die Hydroxygruppe(n) an primäre oder sekundäre Kohlenstoffatome der Kette gebunden sein können und weiter die Alkylkette auch durch Sauerstoff unterbrochen sein kann, und/oder
- $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
- Mono- und/oder Oligoglycosylreste,

mit der Maßgabe, dass mindestens 4 Reste aus $R^1$ bis $R^7$ stehen für OH und dass im Molekül mindestens 2 Paare benachbarter Gruppen -OH vorliegen,

- oder $R^2$, $R^5$ und $R^6$ für OH und die Reste $R^1$, $R^3$, $R^4$ und $R^{7-10}$ für H stehen,

wie sie in der älteren Deutschen Patentanmeldung DE 10244282.7 beschrieben sind.

Vorteile der erfindungsgemäßen Zusammensetzungen enthaltend mindestens ein Antioxidans sind dabei neben den oben genannten Vorteilen insbesondere die antioxidante Wirkung und die gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der Verbindungen nach Formel II, welches sich im DPPH-Assay in einer hohen Kapazität Radikale zu fangen ($EC_{50}$), einer zeitverzögerten Wirkung ($T_{EC50}$ > 120 min) und damit einer mittleren bis hohen antiradikalischen Effizienz (AE) äußert. Zudem vereinigen die Verbindungen nach Formel II im Molekül antioxidative Eigenschaften mit UV-Absorption im UV-A- und/oder-B-Bereich. Bevorzugt sind daher auch Zubereitungen enthaltend zumindest eine Verbindung der Formel II, die dadurch gekennzeichnet ist, dass mindestens zwei benachbarte Reste der Reste $R^1$ bis $R^4$ stehen für OH und mindestens zwei benachbarte Reste der Reste $R^5$ bis $R^7$ stehen für OH. Insbesondere bevorzugte Zubereitungen enthalten zumindest eine Verbindung der Formel II, die dadurch gekennzeichnet ist, dass mindestens drei benachbarte Reste der Reste $R^1$ bis $R^4$ stehen für OH, wobei vorzugsweise die Reste $R^1$ bis $R^3$ für OH stehen.

Als Flavon-Derivate werden erfindungsgemäß Flavonoide und Coumaranone verstanden. Als Flavonoide werden erfindungsgemäß die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden

hierunter jedoch auch die Aglykone, d.h. die zuckerfreien Bestandteile, und die Derivate der Flavonoide und der Aglykone verstanden. Weiterhin wird im Rahmen der vorliegenden Erfindung unter dem Begriff Flavonoid auch Anthocyanidin (Cyanidin) verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavonoide sind vorzugsweise ausgewählt aus folgenden Verbindungen: 4,6,3',4'-Tetrahydroxyauron, Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin), α-Glycosylrutin, Tilirosid sowie deren Sulfaten und Phosphaten. Unter den Flavonoiden sind als erfindungsgemäße Aktivstoffe insbesondere Rutin, Tilirosid, α-Glycosylrutin und Troxerutin bevorzugt.

Unter den Coumaranonen ist 4,6,3',4'-Tetrahydroxybenzylcoumaranon-3 bevorzugt.

Unter Chromon-Derivaten werden vorzugsweise bestimmte Chromen-2-on-Derivate, die sich als Wirkstoffe zur vorbeugenden Behandlung von menschlicher Haut und menschlicher Haare gegen Alterungsprozesse und schädigende Umwelteinflüssen eignen, verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der Formel III

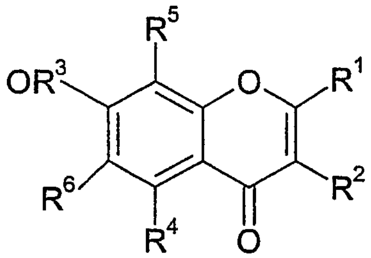

III

wobei

$R^1$ und $R^2$ gleich oder verschieden sein können und ausgewählt sind aus

- H, -C(=O)-$R^7$, -C(=O)-O$R^7$,
- geradkettigen oder verzweigten $C_1$- bis $C_{20}$-Alkylgruppen,
- geradkettigen oder verzweigten $C_3$- bis $C_{20}$-Alkenylgruppen, geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,

$R^3$ steht für H oder geradkettige oder verzweigte $C_1$- bis $C_{20}$-Alkylgruppen, $R^4$ steht für H oder O$R^8$,
$R^5$ und $R^6$ gleich oder verschieden sein können und ausgewählt sind aus

- -H, -OH,
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und

$R^7$ steht für H, geradkettige oder verzweigte $C_1$- bis $C_{20}$-Alkylgruppen, eine Polyhydroxy-Verbindung, wie vorzugsweise einen Ascorbinsäurerest oder glycosidische Reste und
$R^8$ steht für H oder geradkettige oder verzweigte $C_1$- bis $C_{20}$-Alkylgruppen, wobei mindestens 2 der Substituenten $R^1$, $R^2$, $R^4$-$R^6$ verschieden von H sind oder mindestens ein Substituent aus $R^1$ und $R^2$ für -C(=O)-$R^7$ oder - C(=O)-O$R^7$ steht.

Der Anteil an einer oder mehreren Verbindungen ausgewählt aus Flavonoiden, Cromon-Derivaten und Coumaranonen in der erfindungsgemäßen Zubereitung beträgt vorzugsweise von 0.001 bis 5 Gew.-%, besonders bevorzugt von 0.01 bis 2 Gew.-%, bezogen auf die gesamte Zubereitung.

Die erfindungsgemäßen Zubereitungen mit Lichtschutzeigenschaften können darüber hinaus weitere übliche hautschonende oder hautpflegende Wirkstoffe enthalten. Dies können prinzipiell alle dem Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösemitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel IV eingesetzt,

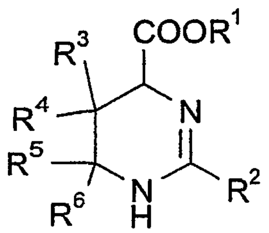

IV

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die Aryloxime, vorzugsweise 2-Hydroxy-5-methyllaurophenonoxim enthalten, zeigen überraschende antiinflammatorische Eignung. Dabei enthalten die Zubereitungen vorzugsweise 0.01 bis 10 Gew.-% des Aryloxims, wobei es insbesondere bevorzugt ist, wenn die Zubereitung 0.05 bis 5 Gew.-% Aryloxim enthält.

Alle hier beschriebenen Verbindungen oder Komponenten, die in den Zubereitungen verwendet werden können, sind entweder bekannt und käuflich erwerbbar oder können nach bekannten Verfahren synthetisiert werden.

Neben den hier beschriebenen Verbindungen können die erfindungsgemäßen Zubereitungen auch mindestens einen Photostabilisator, vorzugsweise entsprechend der Formel V

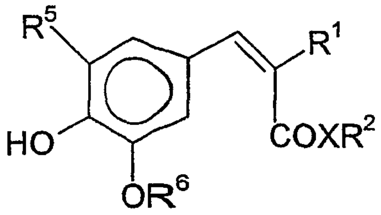

V,

wobei

$R^1$ ausgewählt ist aus $-C(O)CH_3$, $-CO_2R^3$, $-C(O)NH_2$ and $-C(O)N(R^4)_2$;
X is O or NH;
$R^2$ steht für einen linearen oder verzweigten $C_{1-30}$-Alkylrest;
$R^3$ steht für ei nen linearen oder verzweigten $C_{1-20}$-Alkylrest,
alle $R^4$ unabhängig voneinander stehen für H oder lineare oder verzweigte $C_{1-8}$-Alkylreste
$R^5$ steht für H, einen linearen oder verzweigten $C_{1-8}$-Alkylrest oder einen linearen oder verzweigten $-O-C_{1-8}$-Alkylrest und
$R^6$ steht für einen $C_{1-8}$-Alkylrest,

wobei es sich bei dem Photostabilisator insbesondere bevorzugt um 2-(4-Hydroxy-3,5-dimethoxy-benzyliden)-malonsäure-bis-(2-ethyl-hexyl)ester handelt, enthalten. Entsprechende Photostabilisatoren, ihre Herstellung und Verwendung sind in der Internationalen Patentanmeldung WO 03/007906 beschrieben, deren Offenbarung ausdrücklich auch zum Gegenstand der vorliegenden Anmeldung gehört.

Die erfindungsgemäßen Zusammensetzungen können nach Verfahren hergestellt werden, die dem Fachmann gut bekannt sind, insbesondere nach den Verfahren, die zur Herstellung von Öl-in-Wasser-Emulsionen oder Wasser-in-Öl-Emulsionen dienen.

Weitere Gegenstände der vorliegenden Erfindung sind Zubereitungen mit Lichtschutzeigenschaften enthaltend die erfindungsgemäßen UV-Filter in Puderform und einen oder mehrere kosmetisch oder dermatologisch geeignete Träger, ein Verfahren zur Herstellung einer Zubereitung, welches dadurch gekennzeichnet ist, dass mindestens ein erfindungsgemäßes Puder mit einem kosmetisch oder dermatologisch geeigneten Träger vermischt wird, und die Verwendung von erfindungsgemäßen UV-Filtern in Puderform zur Herstellung einer Zubereitung mit Lichtschutzeigenschaften.

Diese Zusammensetzungen können insbesondere in Form von einfachen oder komplizierten Emulsionen (O/W, W/O, O/W/O oder W/O/W), wie Cremes, Milchen, Gelen oder Gel-Cremes, Pulvern und festen Stiften, vorliegen und gegebenenfalls können sie als Aerosole konfektioniert sein und in Form von Schäumen oder Sprays vorliegen. Vorzugsweise liegen diese Zusammensetzungen in Form einer O/W-Emulsion vor.

Die erfindungsgemäßen kosmetischen Zusammensetzungen können als Zusammensetzungen zum Schutz der menschlichen Epidermis oder der Haare gegen UV-Strahlung, als Sonnenschutzmittel oder Schminkprodukte verwendet werden.

Es soll darauf hingewiesen werden, dass in den erfindungsgemäßen Formulierungen zum Sonnenschutz, die einen Träger vom Typ einer Öl-in-Wasser-Emulsion aufweisen, die wässerige Phase (die insbesondere die hydrophilen Filter enthält) im Allgemeinen 50 bis 95 Gew.-% und vorzugsweise 70 bis 90 Gew.-%, bezogen auf die gesamte Formulierung, die Ölphase (die insbesondere die lipophilen Filter enthält) 5 bis 50 Gew.-% und vorzugsweise 10 bis 30 Gew.-%, bezogen auf die gesamte Formulierung und der (Co)emulgator oder die (Co)emulgatoren 0.5 bis 20 Gew.-% und vorzugsweise 2 bis 10 Gew.-%, bezogen auf die gesamte Formulierung, ausmachen.

Geeignet sind Zubereitungen für eine äußerliche Anwendung, beispielsweise als Creme, Lotion, Gel oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösevermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösemittel, Lösevermittler und Emulgatoren, z.B. Wasser, Ethanol, Isopropanol, Ethylcarbonat, Ethlyacetat, Benzylalkohol, Benzylbenzoat, Propylenglykol, 1.3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren. Salze von Fettsäurehalbestern, Fettsäureeiweißhydrolysaten, Isothionate. Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl od er mit Fettsäuren;
- Silikonöle, wie z.B. Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisono-

nanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ d er Formulierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

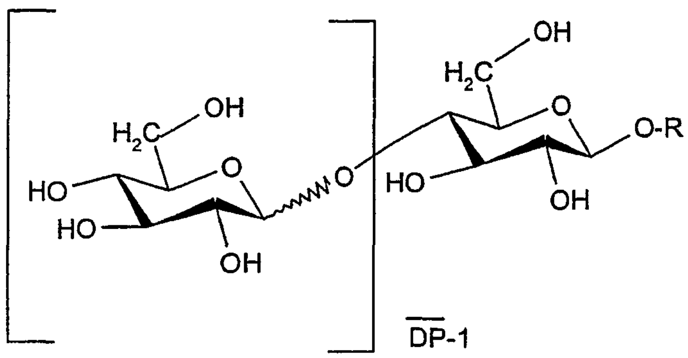

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ bis $p_i$ die Anteile der einfach, zweifach dreifach bis i-fach glucosylierten Produkte in Gewichtsprozenten dar. Erfindungsgemäß vorteilhaft werden Produkte mit Glucosylierungsgraden von 1-2, insbesondere vorteilhaft von 1. 1 bis 1.5. ganz besonders vorteilhaft von 1.2-1.4, insbesondere von 1.3 gewählt.

Der Wert $\overline{DP}$ trägt dem Umstande Rechnung, dass Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werde n gewählt aus der Gruppe Octylglucopyranosid, Nonylglucopyranosid, Decylglucopyranosid, Undecylgluco pyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

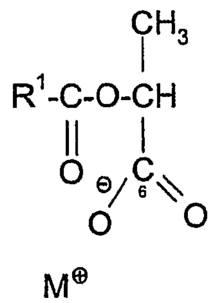

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumi sostearyllactylat, beispielsweise das Produkt Pathionic® ISL von der Gesellschaft American Ingredients Company.

Die Betaine werden vorteilhaft gewäh lt aus der Gruppe der Substanzen, welche sich durch die Strukturformel

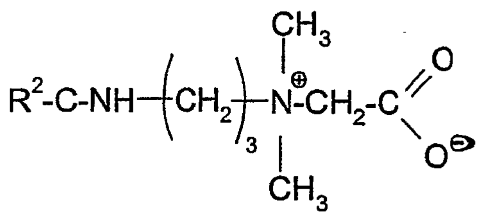

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Caprami dopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0.01-20 Gew.-% bevorzugt 0.05-10 Gew.-%, besonders bevorzugt 0.1-5 Gew.-%, jeweils bezogen auf das Gesamtgewicht der Zusammensetzung, vorliegt oder vorliegen.

Zu Anwendung werden die erfindungsgemäßen, kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol dar stellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices

wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Als erfindungsgemäß besonders bevorzugter Emulgator für O/W-Emulsionen hat sich das Handelsprodukt Ceralution C der Firma Sasol erwiesen.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14.5-15.5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(1 4)stearylether (Steareth-14), Polyethylenglycol(15)stearylether (Steareth-15), Polyethylenglycol(16)stearylether (Steareth-16), Polyethylenglycol(1 7)stearylether (Steareth-17),Polyethylenglycol(18) stearylether(Steareth-18), Polyethylenglycol(19)stearylether (Steareth-19), Polyethylenglycol(2 0)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)-isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol (20)-isostearylether (Isosteareth-20), Polyethylenglycol(13)cetylether (Ceteth-13), Polyethylenglycol(14)cetylether (Ceteth-14), Polyethylenglycol(15)-cetylether (Ceteth-15), Polyethylenglycol(16)cetylether (Ceteth-16), Polyethylenglycol (17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14) isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol-(19)isocetylether (Isoceteth-19), Polyethylenglycol(20)isocetylether (Isoceteth-20), Polyethylenglycol(12) oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)isolaurylether (Isolaureth-12), Polyethylenglycol(13)cetylstearylether (Ceteareth-13), Polyethylenglycol(14)cetylstearylether (Ceteareth-14), Polyethylenglycol(15)cetyl-stearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat, Polyethylenglycol(22)stearat, Polyethylenglycol(23) stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23) isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt

werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen UV-induzierte Alterungsprozesse so wie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder-milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shampoonieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösemittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die nachfolgenden Beispiele sollen die Erfindung näher erläutern, ohne sie jedoch zu beschränken.

**Beispiele:**

**Beispiel 1: Sprühtrocknung mit Zweistoffdüse**

Der Sprühtrockner mit Zweistoffdüse (Typ Mobile Miono 2000 D der Fa. Niro) wird auf einen Eintrittstemperatur von 150°C und eine Austrittstemperatur von 75°C aufgeheizt. Zur Stabilisierung des Systems wird über einen Zeitraum von 10 Minuten Wasser eingesprüht, danach werden 5 kg einer Dispersion von Eusolex® UV-Pearls OMC (Fa. Merck KGaA) in Wasser (Feststoffgehalt 40 Gew.-%) versprüht. Nach 30 Minuten wird der Produktvorlauf entnommen, nach 3.5 Stunden ist die gesamte Dispersion versprüht. Für ca. 15 Minuten wird mit Wasser nachgesprüht. Es werden 1640 g Sprühgut erhalten.

Verfahrensparameter:

| Zweistoffdüse (Zerstäubungsluftmenge 5kg/h (0.3 bar) | |
|---|---|
| Gegenstrombetrieb Produktaustrag: | Zweipunktabscheidung (Trocknerkonus & Zyklon) |
| Trocknungsluftmenge: | 85 kg/h |
| Abgastemperatur: | 50°C |
| Speisemenge Dispersion: | 1.2 kg/h (Förderung mittels Schlauchpumpe) |

**Beispiel 2: Variante mit Zerstäuberrad**

Der Sprühtrockner mit Zerstäuberrad (Betriebsdruck 4.6 bar, 25000 U/min) wird auf einen Eintrittstemperatur von 150°C und eine Austrittstemperatur von 70°C aufgeheizt. Zur Stabilisierung des Systems wird über einen Zeitraum von 10 Minuten Wasser eingesprüht, danach werden 5 kg einer Dispersion von Eusolex® UV-Pearls OMC (Fa. Merck KGaA) in Wasser (Feststoffgehalt 40 Gew.-%) versprüht. Nach 30 Minuten wird der Produktvorlauf entnommen, nach 3.5 Stunden ist die gesamte Dispersion versprüht. Für ca. 15 Minuten wird mit Wasser nachgesprüht. Es werden 1960 g Sprühgut erhalten.

Verfahrensparameter:

| Zerstäuberrad | |
|---|---|
| Gleichstrombetrieb Produktaustrag: | Zweipunktabscheidung (Trocknerkonus & Zyklon) |
| Trocknungsluftmenge: | 85 kg/h |
| Abgastemperatur: | 40°C |
| Speisemenge Dispersion: | 1.2 kg/h (Förderung mittels Schlauchpumpe) |

**Beispielrezeptur Gesichtspuder:**

| **Rohstoff** | **INCI** | **[%]** |
|---|---|---|
| **A** | | |
| Produkt aus Beispiel 1 oder 2 | ETHYLHEXYL METHOXYCINNAMATE, SILICA, PVP, CHLORPHENESIN BHT, AQUA (WATER) | 5,00 |
| Microna® Matte Yellow | MICA, CI 77492 (IRON OXIDES) | 2,60 |
| Microna® Matte Red | MICA, CI 77491 (IRON OXIDES) | 0,70 |
| Microna® Matte Orange | MICA, CI 77491 (IRON OXIDES) | 0,80 |
| Microna® Matte Black | MICA, CI 77499 (IRON OXIDES) | 0,30 |

(fortgesetzt)

| Rohstoff | INCI | [%] |
|---|---|---|
| **A** | | |
| Magnesiumstearat | MAGNESIUM STEARATE | 2,00 |
| Satin Mica | MICA | 15,00 |
| Talkum | TALC | 69,60 |
| **B** | | |
| Ceraphyl 368 | ETHYLHEXYL PALMITATE | 3,92 |
| Propyl-4-hydroxybenzoat | PROPYLPARABEN | 0,08 |

**Herstellung:**

Die Bestandteile der Pudergrundlage werden in den Mixer (La Moulinette von Moulinex) gegeben und 2 mal 10 Sekunden gemixt. Die Mischung wird in ein Becherglas gegeben, der Binder hinzugetropft und mit dem Spatel verrührt. Die Mischung wird wiederum in den Mixer gegeben und 3 mal 10 Sekunden gemixt. Das Puder wird bei 20 bar verpresst.

**Vergleichsbeispiel Gesichtspuder:**

| Rohstoff | INCI | [%] |
|---|---|---|
| **A** | | |
| Microna® Matte Yellow | MICA, CI 77492 (IRON OXIDES) | 2,60 |
| Microna® Matte Red | MICA, CI 77491 (IRON OXIDES) | 0,70 |
| Microna® Matte Orange | MICA, CI 77491 (IRON OXIDES) | 0,80 |
| Microna® Matte Black | MICA, CI 77499 (IRON OXIDES) | 0,30 |
| Magnesiumstearat | MAGNESIUM STEARATE | 2,00 |
| Satin Mica | MICA | 15,00 |
| Talkum | TALC | 74,60 |
| **B** | | |
| Eusolex® 2292 | ETHYLHEXYL METHOXYCINNAMATE, BHT | 2,40 |
| Ceraphyl 368 | ETHYLHEXYL PALMITATE | 1,52 |
| Propyl-4-hydroxybenzoat | PROPYLPARABEN | 0,08 |

**Herstellung:**

Die Bestandteile der Pudergrundlage werden in den Mixer (La Moulinette von Moulinex) gegeben und 2 mal 10 Sekunden gemixt. Die Mischung wird in ein Becherglas gegeben, der Binder hinzugetropft und mit dem Spatel verrührt. Die Mischung wird wiederum in den Mixer gegeben und 3 mal 10 Sekunden gemixt. Das Puder wird bei 20 bar verpresst.

Bei Verwendung des erfindungsgemäßen Puders ergibt sich eine freiere Formulierbarkeit gegenüber der aus dem Vergleichsbeispiel, bei dem ein Teil des Binders durch den flüssigen UV-Filter ersetzt werden muss. Hierdurch kann es zur Einschränkung bei der Formulierung z.B. hinsichtlich des Hautgefühls kommen.

**Beispielrezeptur Hydrogel:**

**Tagespflege für fettige Haut Ölfrei, SPF (DIFFEY) 7, UVA-PF 3**

| Rohstoff | INCI | [%] |
|---|---|---|

(fortgesetzt)

| | | |
|---|---|---|
| **A** | | |
| **Produkt aus Beispiel 1 oder 2** | ETHYLHEXYL METHOXYCINNAMATE, SILICA, PVP, CHLORPHENESIN, BHT, AQUA (WATER), | 8,00 |
| Hispagel 200 | GLYCERIN, GLYCERYL POLYACRYLATE | 25,00 |
| RonaCare® Ectoin | ECTOIN | 0,50 |
| Wasser, demineralisiert | AQUA (WATER) | 65,80 |
| Germaben II | PROPYLENE GLYCOL, DIAZOLIDINYL UREA, METHYLPARABEN,PROPYLPARABEN | 0,70 |

**Herstellung:**

Hispagel vorlegen. Ectoin in Wasser lösen, restliche Bestandteile zugeben und unter Rühren zum Hispagel geben.
Rühren bis eine homogene Mischung entstanden ist.

**Bemerkungen:**

pH (25°C) = 5,5
Viskosität (Brookfield RVD II, Helipath Spindel C, 10 upm, 25°C) = 45.900 cps

**Beispielrezeptur O/W (Einarbeitung in die Wasserphase):**

**O/W Sonnenschutzlotion SPF (in vitro, Diffey Methode) 22 ± 4, UVA-PF 13 ± 3**

| Rohstoff | INCI | [%] |
|---|---|---|
| **A** | | |
| Eusolex® 6300 | 4-METHYLBENZYLIDENE CAMPHOR | 4,00 |
| Eusolex® 9020 | BUTYL METHOXYDIBENZOYLMETHANE | 5,00 |
| Hostacerin DGI | POLYGLYCERYL-2SESQUIISOSTEARATE | 4,00 |
| Cetiol B | DIBUTYL ADIPATE | 4,00 |
| Crodamol DOA | DIOCTYL ADIPATE | 2,00 |
| Tegosoft TN | C12-15 ALKYL BENZOATE | 4,00 |
| Eutanol G | OCTYLDODECANOL | 4,50 |
| **B** | | |
| Aristoflex AVC | AMMONIUM ACRYLOYLDIMETYLTAURATE / VP COPOLYMER | 0,50 |
| **C** | | |
| Wasser, demineralisiert | AQUA (WATER) | 40,00 |
| **D** | | |
| Produkt aus Beispiel 1 oder 2 | ETHYLHEXYL METHOXYCINNAMATE, SILICA, PVP, CHLORPHENESIN, BHT AQUA (WATER), | 8,40 |
| Hostapon KCG | SODIUM COCOYL GLUTAMATE | 1,50 |
| Panthenol- D | PANTHENOL | 0,50 |
| Glycerin, wasserfrei | GLYCERIN | 3,00 |
| Wasser, demineralisiert | AQUA (WATER) | 17,40 |

(fortgesetzt)

| | | |
|---|---|---|
| **D** | | |
| **E** | | |
| Phenonip | PHENOXYETHANOL, BUTYLPARABEN, ETHYLPARABEN, PROPYLPARABEN, METHYLPARABEN | 0,70 |
| RonaCare® Tocopherolacetat | TOCOPHERYL ACETATE | 0,50 |
| Parfümöl (q.s.) | PARFUM | 0,00 |

**Herstellung:**

A auf 80°C erhitzen
B in A einrühren
C auf 80°C erwärmen

intermediäre W/O Emulsion herstellen:

C in A/B einrühren mit hoher Schergeschwindigkeit (Ultra Thurrax) für etwa 2 Minuten Kalte Phase D tropfenweise sehr langsam zugeben, bis Phasenumkehr stattfindet, mindestens 2 h kaltrühren
E hinzugeben und nochmals eine Stunde rühren
Homogenisieren.

**Bemerkungen:**

pH (25°C) = 6,0
Viskosität (Brookfield RVD II, Helipath Spindel B, 50 upm, 25°C) = 1.120 cps

**Beispielrezeptur W/O (Einarbeitung in die Ölphase):**

**Complete Sun Protection (W/O) in vitro SPF (Diffey, Transpore, SPF290) = 40+/-9, Lambda krit. (DGK) = 373nm**

| | **Rohstoff** | **INCI** | **[%]** |
|---|---|---|---|
| **A** | | | |
| | Produkt aus Beispiel 1 oder 2 | ETHYLHEXYL METHOXYCINNAMATE SILICA, PVP, CHLORPHENESIN, BHT AQUA (WATER), | 4,00 |
| | Eusolex® 6300 | 4-METHYLBENZYLIDENE CAMPHOR | 1,00 |
| | Eusolex® 9020 | BUTYL METHOXYDIBENZOYLMETHANE | 1,00 |
| | Cremophor WO 7 | PEG-7 HYDROGENATED CASTOR OIL | 6,00 |
| | Elfacos ST 9 | PEG-45 DODECYL GLYCOL COPOLYMER | 2,00 |
| | Jojobaöl | BUXUS CHINENSIS (JOJOBA OIL) | 9,00 |
| | Isopropylmyristat | ISOPROPYL MYRISTATE | 3,50 |
| | Abil 350 | DIMETHICONE | 1,00 |
| **B** | | | |
| | Eusolex® T-AVO | TITANIUM DIOXIDE, SILICA | 5,00 |
| **C** | | | |
| | Glycerin (87 % reinst) | GLYCERIN | 5,00 |

(fortgesetzt)

| | | | |
|---|---|---|---|
| C | | | |
| | Titriplex® III | DISODIUM EDTA | 0,20 |
| | Wasser, demineralisiert | AQUA (WATER) | 61,50 |
| | Germall 115 | IMIDAZOLIDINYL UREA | 0,30 |
| D | | | |
| | Phenonip | PHENOXYETHANOL, BUTYLPARABEN, ETHYLPARABEN, PROPYLPARABEN, METHYLPARABEN | 0,50 |
| | Parfümöl (q.s.) | PARFUM | 0,00 |

**Herstellung:**

Phase A auf 80°C erhitzen, Phase B unter rühren eintragen und 3 Minuten homogenisieren (Zauberstab Stufe 1). Phase C auf 80°C erhitzen und in Phase A/B einhomogenisieren (1 Minute Zauberstab Stufe 1 & 30s Stufe 2. Unter rühren auf 40°C abkühlen, Phase D zugeben und nochmals homogenisieren (MFR-Rührer 1200 upm 1 Minute).

**Bemerkungen:**

Viskosität (Brookfield RVT-DV II, Helipath C, 10 rpm, 24°C) = 11700 mPa s

**Beispielrezeptur O/W (Einarbeitung in die Ölphase):**

**Sonnenschutzlotion, wasserfest (O/W) erwarteter SPF ca. 25**

| **Rohstoff** | | **INCI** | **[%]** |
|---|---|---|---|
| **A** | | | |
| | Produkt aus Beispiel 1 oder 2 | ETHYLHEXYL METHOXYCINNAMATE, BHT SILICA, PVP, CHLORPHENESIN, BHT AQUA (WATER), | 6.00 |
| | Eusolex® 6300 | 4-METHYLBENZYLIDENE CAMPHOR | 1.00 |
| | Eusolex® 9020 | BUTYL METHOXYDIBENZOYLMETHANE | 3.00 |
| | Amphisol A | CETYL PHOSPHATE | 2.00 |
| | Lanette O | CETEARYL ALCOHOL | 0.50 |
| | Cutina GMS | GLYCERYL STEARATE | 4.00 |
| | Dow Corning 200 (100cs) | DIMETHICONE | 0.50 |
| | Crodamol AB | C12-15 ALKYL BENZOATE | 9.00 |
| | Antaron WP-660 | TRICONTANYL PVP | 3.00 |
| **B** | | | |
| | Eusolex® 232 | PHENYLBENZIMIDAZOLE SULFONIC ACID | 2.00 |
| | Tris(hydroxymethyl)-aminomethan | TROMETHAMINE | 0.90 |
| | Wasser, demineralisiert | AQUA (WATER) | 62.20 |
| | Carbopol 980, 2% solution, neutralisiert auf pH 7 | AQUA, CARBOMER | 5.00 |
| **C** | | | |
| | Parfum (q.s.) | PARFUM | 0.20 |
| | Germaben II | PROPYLENE GLYCOL, DIAZOLIDINYL UREA, METHYLPARABEN,PROPYLPARABEN | 0.70 |

(fortgesetzt)

| | | |
|---|---|---|
| **C** | | |
| Natriumhydroxid, 10% Lösung | SODIUM HYDROXIDE | 0.00 |

**Herstellung:**

Zur Neutralisierung von Eusolex®232 wird Tris(hydroxymethyl)-aminomethan im Wasser von Phase B gelöst und unter Rühren mit Eusolex® 232 versetzt. Restliche Inhaltsstoffe von Phase B gleichmäßig zugeben und auf 80°C erhitzen. Phase A wird auf 75°c erhitzt. Phase B wird unter leichtem Rühren langsam zu Phase A gegeben und einhomogenisiert. Unter Rühren auf 40°C abkühlen, Phase C zugeben und pH auf 7 einstellen.

**Bemerkungen:**

pH (25°C) = 6.9
Viskosität (Brookfield RVT-DV II, Helipath C, 10 rpm, 25°C) = 55,000 cps

**Beispielrezeptur W/O (Einarbeitung in die Wasserphase):**

**Sonnenschutz Spray-Lotion (W/O)**

| Rohstoff | INCI | [%] |
|---|---|---|
| **A** | | |
| Arlacel P135 | PEG-30 DIPOLYHYDROXYSTEARATE | 3.00 |
| Cetiol A | HEXYL LAURATE | 5.50 |
| Arlamol HD | ISOHEXADECANE | 8.00 |
| Miglyol 812 N | CAPRYLIC/CAPRIC TRIGLYCERIDE | 4.00 |
| Arlamol DOA | DIOCTYL ADIPATE | 4.00 |
| **B** | | |
| Produkt aus Beispiel 1 oder 2 | ETHYLHEXYL METHOXYCINNAMATE, SILICA, PVP, CHLORPHENESIN, BHT AQUA (WATER), | 8.00 |
| RonaCare® Ectoin | ECTOIN | 0.10 |
| Magnesiumsulfat heptahydrat | MAGNESIUM SULFATE | 0.70 |
| Glycerol (etwa 87%) | GLYCERIN | 3.00 |
| Titriplex® III | DISODIUM EDTA | 0.05 |
| Wasser, demineralisiert | AQUA (WATER) | 57.65 |
| **C** | | |
| Eusolex® T-S | TITANIUM DIOXIDE, ALUMINA, STEARIC ACID | 5.00 |
| **D** | | |
| Phenonip | PHENOXYETHANOL, BUTYLPARABEN, ETHYLPARABEN, PROPYLPARABEN,METHYLPARABEN | 0.70 |
| Fragrance L'EAU D'ETE +D12921CT | PARFUM | 0.30 |

**Herstellung:**

Phase A und B werden separat kombiniert und auf 80°C erhitzt. Phase B wird langsam zu Phase A und kräftigem Rühren gegeben und einhomogenisiert. Unter Rühren auf 40°C abkühlen, Phase C zugeben. Phase D nach homogener Dispergierung von Eusolex® T-S zugeben und unter Rühren abkühlen.

**Bemerkungen:**

Viskosität 5,000 mPas (Brookfield LV, Spindle 4; 12 rpm).

**Sonnenschutzpflegelotion:**

| Rohstoff | INCI | [%] |
|---|---|---|
| **A** | | |
| Paraffin dickflüssig | PARAFFINUM LIQUIDUM | 6,50 |
| Pelemol BIP | ISOPROPYLPHTALIMIDE, BUTYLPHTALIDE | 6,00 |
| Isopropylpalmitat | ISOPROPYL PALMITATE | 7,50 |
| Sojaöl | GLYCINE SOJA | 5,00 |
| RonaCare® Tocopherolacetat | TOCOPHEROLACETAT | 1,00 |
| Carbopol Ultrez 10 | CARBOMER | 0,30 |
| **B** | | |
| Produkt aus Beispiel 1 oder 2 | ETHYLHEXYL METHOXYCINNAMATE, SILICA, PVP, CHLORPHENESIN, BHT AQUA (WATER) | 5,60 |
| Produkt aus Beispiel 1 oder 2 | HOMOSALATE, BUTYLMETHOXY DIBENZOYLMETHANE, DIETHYLHEXYL SYRINGYLIDENE MALONATE SILICA, PVP, CHLORPHENESIN, BHT AQUA | 5,60 |
| Wasser, demineralisiert | AQUA (WATER) | 54,20 |
| Sisterna L70-C | SUCROSE LAURATE , AQUA, ALCOHOL | 6,00 |
| Phenonip | PHENOXYETHANOL, BUTYLPARABENE, METHYLPARABENE, PROPYLPARABENE, ETHYLPARABENE | 1,00 |
| **C** | | |
| Natronlauge, 10 %ig | SODIUM HYDROXIDE,AQUA (WATER) | 1,20 |
| **D** | | |
| Parfümöl (q.s.) | PARFUME, FRAGANCE | 0,30 |
| | | 100,00 |

**Herstellung:**

Phase A bis auf Carbopol zusammengeben. Falls nötig, auf ca. 50°C erwärmen. Carbopol einarbeiten und vorgelöste Phase B unter Rühren einemulgieren. Homogenisieren. Nach Zugabe von Phase C nochmals kurz homogenisieren Phase D zugeben

## Patentansprüche

1. UV-Filter in Puderform, erhältlich durch Sprühtrocknung einer Dispersion enthaltend UV-Filter, **dadurch gekennzeichnet, dass** die UV-Filter verkapselte organische UV-Filter sind, wobei die Wände der Kapseln anorganisch sind.

2. UV-Filter in Puderform gemäß Anspruch 1, **dadurch gekennzeichnet, dass** die Dispersion wässrig ist.

3. UV-Filter in Puderform gemäß Anspruch 1, **dadurch gekennzeichnet, dass** die Wände der Kapseln aus Kieselgel oder Siliciumdioxid aufgebaut sind.

4. UV-Filter gemäß Anspruch 1, **dadurch gekennzeichnet, dass** die Kapseln weitere Substanzen enthalten.

**5.** UV-Filter gemäß Anspruch 4, **dadurch gekennzeichnet, dass** es sich bei den Substanzen um Photostabilisatoren, kosmetische Öle und/oder Antioxidantien handelt.

**6.** UV-Filter in Puderform gemäß einem der Ansprüche 1 bis 5, **dadurch gekennzeichnet, dass** das Puder zusätzlich nachbehandelt ist.

**7.** Verfahren zur Herstellung von UV-Filtern in Puderform gemäß Anspruch 1, **dadurch gekennzeichnet, dass** Dispersionen von verkapselten organischen UV-Filtern sprühgetrocknet werden.

**8.** Verfahren nach Anspruch 7, **dadurch gekennzeichnet, dass** die UV-Filter in partikulärer Form vorliegen.

**9.** Verfahren nach Anspruch 8, **dadurch gekennzeichnet, dass** die Größe der UV-Filter in partikulärer Form 10 nm bis 100 $\mu$m beträgt.

**10.** Verfahren nach einem der Ansprüche 7 bis 9, **dadurch gekennzeichnet, dass** vor oder während des Verfahrens Additive eingebracht werden.

**11.** Verfahren nach einem der Ansprüche 7 bis 10, **dadurch gekennzeichnet, dass** die UV-Filter in Pulverform nachbehandelt werden.

**12.** Zubereitungen mit Lichtschutzeigenschaften enthaltend UV-Filter in Puderform gemäß Anspruch 1.

**13.** Zubereitung nach Anspruch 12, **dadurch gekennzeichnet, dass** es sich um eine topisch anwendbare Zubereitung handelt.

**14.** Zubereitung nach Anspruch 12, **dadurch gekennzeichnet, dass** es sich um eine Zubereitung für die dekorative Kosmetik handelt.

**15.** Zubereitung mit Lichtschutzeigenschaften nach mindestens einem der vorstehenden Ansprüche, **dadurch gekennzeichnet, dass** die Zubereitung zusätzlich mindestens einen organischen und/oder anorganischen UV-Filter enthält.

**16.** Zubereitung mit Lichtschutzeigenschaften nach mindestens einem der vorstehenden Ansprüche, **dadurch gekennzeichnet, dass** die Zubereitung mindestens einen Selbstbräuner enthält.

**17.** Zubereitung mit Lichtschutzeigenschaften nach mindestens einem der vorhergehenden Ansprüche, **dadurch gekennzeichnet, dass** die Zubereitung mindestens einen Photostabilisator enthält.

**18.** Zubereitung nach Anspruch 17, **dadurch gekennzeichnet, dass** der Photostabilisator ausgewählt ist aus Verbindungen entsprechend der Formel V

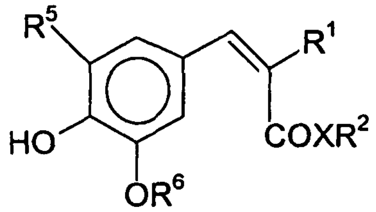

V,

wobei

$R^1$ ausgewählt ist aus $-C(O)CH_3$, $-CO_2R^3$, $-C(O)NH_2$ and $-C(O)N(R^4)_2$; X is O or NH-,
$R^2$ steht für einen linearen oder verzweigten $C_{1-30}$-Alkylrest;
$R^3$ steht für einen linearen oder verzweigten $C_{1-20}$-Alkylrest,
alle $R^4$ unabhängig voneinander stehen für H oder lineare oder verzweigte $C_{1-8}$-Alkylreste
$R^5$ steht für H, einen linearen oder verzweigten $C_{1-8}$-Alkylrest oder einen linearen oder verzweigten $-O-C_{1-8}$-Alkylrest und
$R^6$ steht für einen $C_{1-8}$-Alkylrest.

19. Zubereitung mit Lichtschutzeigenschaften nach mindestens einem der vorhergehenden Ansprüche, **dadurch gekennzeichnet, dass** die Zubereitung einen oder mehrere weitere UV-Filter enthält, die ausgewählt sind aus der Gruppe bestehend aus 3-(4'-Methylbenzyliden)-dl-kampfer, Methoxyzimtsäureoctylester, 3,3,5-Trimethyl-cyclohexylsalicylat, 4-(Dimethylamino)benzoesäure2-ethylhexylester, 2-Cyano-3,3-diphenylacrylsäure-2-ethylhexylester, 2-Phenyl-benzimidazol-5-sulfonsäure sowie ihren Kalium-, Natrium- und Triethanolaminsalzen.

20. Zubereitung mit Lichtschutzeigenschaften nach mindestens einem der vorhergehenden Ansprüche geeignet zum Schutz von Körperzellen gegen oxidativen Stress, **dadurch gekennzeichnet, dass** sie vorzugsweise ein oder mehrere Antioxidantien enthält.

21. Zubereitung mit Lichtschutzeigenschaften nach mindestens einem der vorstehenden Ansprüche, **dadurch gekennzeichnet, dass** es sich um eine Emulgator-freie Emulsion handelt.

22. Verfahren zur Herstellung einer Zubereitung, **dadurch gekennzeichnet, dass** ein UV-Filter nach Anspruch 1 mit einem kosmetisch oder dermatologisch geeignetem Träger und ggf. weiteren Inhaltstoffen vermischt wird.

## Claims

1. UV filters in powder form, obtainable by spray-drying a dispersion comprising UV filters, **characterised in that** the UV filters are encapsulated organic UV filters, where the walls of the capsules are inorganic.

2. UV filters in powder form according to Claim 1, **characterised in that** the dispersion is aqueous.

3. UV filters in powder form according to Claim 1, **characterised in that** the walls of the capsules are built up from silica gel or silicon dioxide.

4. UV filters according to Claim 1, **characterised in that** the capsules comprise further substances.

5. UV filters according to Claim 4, **characterised in that** the substances are photostabilisers, cosmetic oils and/or antioxidants.

6. UV filters in powder form according to one of Claims 1 to 5, **characterised in that** the powder has additionally been after-treated.

7. Process for the preparation of UV filters in powder form according to Claim 1, **characterised in that** dispersions of encapsulated organic UV filters are spray-dried.

8. Process according to Claim 7, **characterised in that** the UV filters are in particulate form.

9. Process according to Claim 8, **characterised in that** the size of the UV filters in particulate form is 10 nm to 100 μm.

10. Process according to one of Claims 7 to 9, **characterised in that** additives are introduced before or during the process.

11. Process according to one of Claims 7 to 10, **characterised in that** the UV filters in powder form are after-treated.

12. Preparations having light-protection properties comprising UV filters in powder form according to Claim 1.

13. Preparation according to Claim 12, **characterised in that** it is a preparation which can be applied topically.

14. Preparation according to Claim 12, **characterised in that** it is a preparation for decorative cosmetics.

15. Preparation having light-protection properties according to at least one of the preceding claims, **characterised in that** the preparation additionally comprises at least one organic and/or inorganic UV filter.

16. Preparation having light-protection properties according to at least one of the preceding claims, **characterised in that** the preparation comprises at least one self-tanning agent.

17. Preparation having light-protection properties according to at least one of the preceding claims, **characterised in that** the preparation comprises at least one photostabiliser.

18. Preparation according to Claim 17, **characterised in that** the photostabiliser is selected from compounds conforming to the formula V

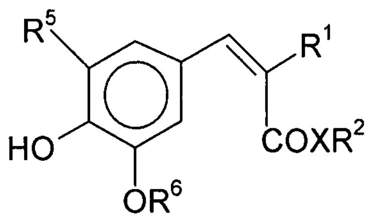

V,

where

$R^1$ is selected from $-C(O)CH_3$, $-CO_2R^3$, $-C(O)NH_2$ and $-C(O)N(R^4)_2$; X is O or NH;
$R^2$ stands for a linear or branched $C_{1-30}$-alkyl radical;
$R^3$ stands for a linear or branched $C_{1-20}$-alkyl radical,
all $R^4$, independently of one another, stand for H or linear or branched $C_{1-8}$-alkyl radicals
$R^5$ stands for H, a linear or branched $C_{1-8}$-alkyl radical or a linear or branched $-O-C_{1-8}$-alkyl radical and
$R^6$ stands for a $C_{1-8}$-alkyl radical.

19. Preparation having light-protection properties according to at least one of the preceding claims, **characterised in that** the preparation comprises one or more further UV filters selected from the group consisting of 3-(4'-methylbenzylidene)-dl-camphor, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid, and the potassium, sodium and triethanolamine salts thereof.

20. Preparation having light-protection properties according to at least one of the preceding claims, suitable for the protection of body cells against oxidative stress, **characterised in that** it preferably comprises one or more antioxidants.

21. Preparation having light-protection properties according to at least one of the preceding claims, **characterised in that** it is an emulsifier-free emulsion.

22. Process for the preparation of a preparation, **characterised in that** a UV filter according to Claim 1 is mixed with a cosmetically or dermatologically suitable vehicle and optionally further ingredients.

**Revendications**

1. Filtres anti-UV sous forme de poudre, pouvant être obtenus par séchage par pulvérisation d'une dispersion comprenant des filtres anti-UV, **caractérisés en ce que** les filtres anti-UV sont des filtres anti-UV organiques encapsulés, où les parois des capsules sont inorganiques.

2. Filtres anti-UV sous forme de poudre selon la revendication 1, **caractérisés en ce que** la dispersion est aqueuse.

3. Filtres anti-UV sous forme de poudre selon la revendication 1, **caractérisés en ce que** les parois des capsules sont constituées de gel de silice ou de dioxyde de silicium.

4. Filtres anti-UV selon la revendication 1, **caractérisés en ce que** les capsules comprennent d'autres substances.

5. Filtres anti-UV selon la revendication 4, **caractérisés en ce que** les substances sont des agents photostabilisants, des huiles cosmétiques et/ou des antioxydants.

6. Filtres anti-UV sous forme de poudre selon l'une des revendications 1 à 5, **caractérisés en ce que** la poudre a subi

de plus un post-traitement.

7. Procédé de préparation de filtres anti-UV sous forme de poudre selon la revendication 1, **caractérisé en ce que** des dispersions de filtres anti-UV organiques encapsulés sont séchées par pulvérisation.

8. Procédé selon la revendication 7, **caractérisé en ce que** les filtres anti-UV se trouvent sous forme particulaire.

9. Procédé selon la revendication 8, **caractérisé en ce que** la taille des filtres anti-UV sous forme particulaire va de 10 nm à 100 $\mu$m.

10. Procédé selon l'une des revendications 7 à 9, **caractérisé en ce que** des additifs sont introduits avant ou pendant le procédé.

11. Procédé selon l'une des revendications 7 à 10, **caractérisé en ce que** les filtres anti-UV sous forme de poudre subissent un post-traitement.

12. Préparations ayant des propriétés photo-protectrices, comprenant des filtres anti-UV sous forme de poudre selon la revendication 1.

13. Préparation selon la revendication 12, **caractérisée en ce qu'**il s'agit d'une préparation pouvant être appliquée par voie topique.

14. Préparation selon la revendication 12, **caractérisée en ce qu'**il s'agit d'une préparation pour des produits cosmétiques décoratifs.

15. Préparation ayant des propriétés photo-protectrices selon au moins l'une des revendications précédentes, **caractérisée en ce que** la préparation comprend en outre au moins un filtre anti-UV organique et/ou inorganique.

16. Préparation ayant des propriétés photo-protectrices selon au moins l'une des revendications précédentes, **caractérisée en ce que** la préparation comprend au moins un agent auto-bronzant.

17. Préparation ayant des propriétés photo-protectrices selon au moins l'une des revendications précédentes, **caractérisée en ce que** la préparation comprend au moins un photostabilisant.

18. Préparation selon la revendication 17, **caractérisée en ce que** le photostabilisant est choisi parmi des composés répondant à la formule V

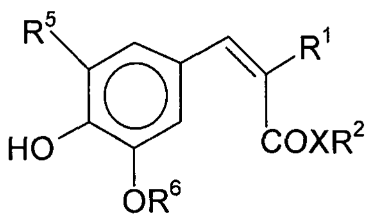

V,

dans laquelle

$R^1$ est choisi parmi $-C(O)CH_3$, $-CO_2R^3$, $-C(O)NH_2$ et $-C(O)N(R^4)_2$ ; X est O ou NH ;
$R^2$ représente un radical $C_{1-30}$-alkyle linéaire ou ramifié ;
$R^3$ représente un radical $C_{1-20}$-alkyle linéaire ou ramifié ;
tous les $R^4$, indépendamment les uns des autres, représentent H ou des radicaux $C_{1-8}$-alkyle linéaires ou ramifiés ;
$R^5$ représente H, un radical $C_{1-8}$-alkyle linéaire ou ramifié ou un radical $-O-C_{1-8}$-alkyle linéaire ou ramifié ; et
$R^6$ représente un radical $C_{1-8}$-alkyle.

19. Préparation ayant des propriétés photo-protectrices selon au moins l'une des revendications précédentes, **caractérisée en ce que** la préparation comprend un ou plusieurs autres filtres anti-UV choisis dans le groupe constitué

par le 3-(4'-méthylbenzylidène)-dl-camphre, le méthoxycinnamate d'octyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique, et les sels de potassium, sodium et triéthanolamine de ceux-ci.

**20.** Préparation ayant des propriétés photo-protectrices selon au moins l'une des revendications précédentes, convenable pour la protection de cellules de l'organisme contre un stress oxydatif, **caractérisée en ce qu'**elle comprend de préférence un ou plusieurs antioxydants.

**21.** Préparation ayant des propriétés photo-protectrices selon au moins l'une des revendications précédentes, **caractérisée en ce qu'**il s'agit d'une émulsion dépourvue d'émulsifiants.

**22.** Procédé de préparation d'une préparation, **caractérisé en ce qu'**un filtre anti-UV selon la revendication 1 est mélangé avec un véhicule convenable sur le plan cosmétique ou dermatologique et éventuellement d'autres ingrédients.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1382328 A
- WO 0009652 A
- WO 0072806 A
- WO 0071084 A
- US 2003143166 A1
- US 6337089 B
- US 6238650 B
- WO 9304665 A
- EP 0487404 A
- FR 2326405 A
- FR 2440933 A
- EP 0114607 A
- WO 9966896 A
- DE 10244282
- EP 0671161 A
- DE 4116123 A
- WO 03007906 A
- DE OS4308282 A


### In der Beschreibung aufgeführte Nicht-Patentliteratur


- Rowe Colour Index. Society of Dyers and Colourists, 1971
- **K. LEMANSKA ; H. SZYMUSIAK ; B. TYRAKOWSKA ; R. ZIELINSKI ; I.M.C.M. RIETJENS.** *Current Topics in Biophysics,* 2000, vol. 24 (2), 101-108
- **C.A. RICE-EVANS ; N.J. MILLER ; G. PAGANGA.** *Trends in Plant Science,* 1997, vol. 2 (4), 152-159
- **K. LEMANSKA ; H. SZYMUSIAK ; B. TYRAKOWSKA ; R. ZIELINSKI ; A.E.M.F. SOFFERS ; I.M.C.M. RIETJENS.** *Free Radical Biology&Medicine,* 2001, vol. 31 (7), 869-881
- *Römpp Chemie Lexikon,* 1993, vol. 9
- **E. A. GALINSKI et al.** *Eur. J. Biochem.,* 1985, vol. 149, 135-139